# EUROPEAN PATENT SPECIFICATION

(11) **EP 2 528 585 B1**
(45) Date of publication and mention of the grant of the patent: **24.07.2019**
(21) Application number: 11706963.3
(22) Date of filing: 28.01.2011
(51) Int. Cl.: A61K 9/00, A61K 31/381, A61K 31/58, A61K 31/167, A61K 45/06, A61K 31/46, A61K 31/439, A61P 11/08

(54) **PHARMACEUTICAL COMPOSITIONS COMPRISING TIOTROPIUM, FORMOTEROL AND BUDESONIDE**
PHARMAZEUTISCHE ZUSAMMENSETZUNG MIT TIOTROPIUM, FORMOTEROL UND BUDESONID
COMPOSITIONS PHARMACEUTIQUES COMPRENANT DU TIOTROPIUM, DU FORMOTÉROL ET DU BUDÉSONIDE

(30) Priority: 29.01.2010 TR 201000680
(43) Date of publication of application: 05.12.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000019
(87) International publication number: WO 2011/093817

(56) References cited:
- WO-A1-02/49616
- WO-A1-2004/019985
- WO-A1-2005/053646
- WO-A1-2005/053648
- WO-A2-03/000241
- WO-A2-2008/102128
- US-A1- 2009 311 314
- WELTE TOBIAS ET AL: "Efficacy and tolerability of budesonide/formoterol added to tiotropium in patients with chronic obstructive pulmonary disease", AMERICAN JOURNAL OF RESPIRATORY AND CRITICAL CARE MEDICINE, AMERICAN LUNG ASSOCIATION, NEW YORK, NY, US, vol. 180, no. 8, 1 January 2009 (2009-01-01), pages 741-750, XP009147846, ISSN: 1073-449X
- WELTE ET AL: "Optimising treatment for COPD - new strategies for combination therapy", INTERNATIONAL JOURNAL OF CLINICAL PRACTICE, MEDICON INTERNATIONAL, ESHER, GB, vol. 63, no. 8, 1 January 2009 (2009-01-01), pages 1136-1149, XP009147844, ISSN: 1368-5031
- XIAN MING ZENG ET AL: "EFFECTS OF PARTICLE SIZE AND ADDING SEQUENCE OF FINE LACTOSE ON THE DEPOSITION OF SALBUTAMOL SULPHATE FROM A DRY POWDER FORMULATION", INTERNATIONAL JOURNAL OF PHARMACEUTICS, ELSEVIER BV, NL, vol. 182, no. 2, 1 January 1999 (1999-01-01), pages 133-144, XP001030465, ISSN: 0378-5173, DOI: 10.1016/S0378-5173(99)00021-6

## Description

The present invention is related to a pharmaceutical composition comprising tiotropium, formoterol, budesonide and/or pharmaceutically acceptable derivatives thereof and the use of this composition in the treatment of respiratory diseases particularly in asthma, allergic rhinitis and chronic obstructive pulmonary diseases (COPD).

Airways, in other words bronchia, are the channels which function to distribute the inhaled air into the lung tissues. In the case of respiratory diseases such as asthma or chronic obstructive pulmonary disease (COPD), stimulants such as allergen, infection, good and bad smell, smoke, genetic factors and exercise cause contractions in the airway muscles (bronchoconstruction) and/or excessive secretion in glands and results in contractions in the airway. Hence, respiration gets more difficult as the inhaled air cannot be exhaled.

Corticosteroids which are used in the treatment of asthma and COPD are synthetic and strong anti-inflammatory drugs that are similar to natural corticosteroid hormones produced by adrenal glands. They prevent both the transcription of the inflammatory gene and the activation of the anti-inflammatory gene. In addition to this, they increase the transcription of β₂ receptors. Furthermore, corticosteroids prevent the tolerance that develops after a long-term application of β₂ agonists. Beclomathasone, budesonide, ciclesonide, flunisolide, fluticasone are among the steroids that are used for the treatment of respiratory diseases.

Corticosteroids are not preferred in acute asthma crises as they do not have rapid onset of action. Since inhaled corticosteroids may prevent growth in children, they arc advised to be used in the lowest possible amount. Long-term use of corticosteroids may cause cataract and glaucoma. In addition, they may cause some serious side-effects such as osteoporosis, high cholesterol, edema, encepholalgia, weight gain, insomnia and some skin problems.

β₂ adrenergic agonists which are used in the treatment of respiratory diseases such as asthma and COPD affect the muscles around the air vessels by activating β₂ adrenergic receptors. They reduce or eliminate bronchospasm. Bronchodilator β₂-agonists are categorized into two groups as long-acting and short-acting. Short-acting beta-agonists such as salbutamol. levosalbutamol, prosaterol, fenoterol, terbutaline, pirbuterol, metoproterenol, bitolterol mesilate have a rather rapid onset of action such as 3-5 minutes and their duration of action is of 4-6 hours. As they have a rapid onset of action, they are given as relaxant but they should be taken very often since they have a short duration of action. Long-acting β₂ agonists have a slower onset of action compared to the other group but their duration of action is of 12 hours. Salmeterol, formoterol, bambuterol and clenbuterol can be given as examples to long-acting β₂ agonists. Long-acting β₂-agonists are often used in the treatment of patients who present asthma symptoms at nights and in the treatment of asthma stimulated by exercise.

β₂ agonists are known as the most effective agent in order to eliminate acute asthma symptoms. One of the most important factors for their being used as symptomatolytic is their onset of action. Some long-acting β₂ agonists can have the onset of action of short acting β₂ agonists when used at specific doses.

β₂ agonists affect the muscles above the air vessels. However, they might affect the muscles around the heart and the bones. In the case that they affect the heart muscles, acceleration of heartbeat and palpitation might be observed. In 2005, US Food and Drug Administration (FDA) revealed that many long-acting β₂-agonist drugs increase wheezing symptom in patients. Following this, in a study carried out by Cornell and Stanford Universities, it was found out that regular intake of β-agonists in the treatment of COPD increases health problems resulting from respiratory tract.

Anticholinergics are the other active agents which are utilized in the treatment of respiratory diseases. Anticholinergics influence large airways including the muscles above bronchia, while β₂ agonists influence small airways, in other words bronchioles. Anticholinergics such as β₂ agonists are categorized into two groups as long-acting and short-acting. Short-acting anticholinergics containing ipratropium bromide and oxitropium bromide have an onset of action of 15 minutes and their duration of action is 6-8 hours. The long-acting anticholinergic agent is tiotropium. Tiotropium has an onset of action of 20 minutes and its duration of action is 24 hours. Thus, it is enough to take it once a day. Side effects of anticholinergics are weaker than the side effects of β₂ agonists.

The use of combination drugs in the treatment of respiratory diseases such as asthma and COPD is very effective particularly in decreasing asthma attacks. It is possible that the severity or occurrence possibility of the abovementioned side effects decreases as the active substances that are used in combinations are more effective at lower doses compared to the active substances used alone.

However, decreasing the side effects that arise from the active agents is not sufficient to provide effective treatment for respiratory diseases. The medicaments used in the formulations should be selected in a way to give the best combination and furthermore, they should be in the most stable form. Moreover, the compositions comprising them should be formulated in such a way that the composition is stable and also it reaches to the target area in the most efficient way.

The inventor has surprisingly found that unexpected therapeutic benefits are obtained through the use of the combination comprising tiotropium bromide with water content less than or equal to 2.5%. formoterol fumarate and budesonide together for simultaneous or sequential administration in the prevention or treatment of respiratory diseases.

It was seen that a combination comprising tiotropium bromide with water content less than or equal to 2.5%, formoterol fumarate and budesonide provides the most stable and therapeutically beneficial combination for simultaneous or sequential administration in the prevention or treatment of respiratory diseases. Preferably, a composition comprising tiotropium bromide with water content less than or equal to 2.5%, formoterol fumarate and budesonide is administered simultaneously. Formoterol fumarate used in the formulation is preferably in dihydrate form.

In another aspect, said composition comprises tiotropium bromide with water content less than or equal to 2.5%, formoterol fumarate, budesonide and lactose.

In another aspect, tiotropium bromide with water content less than or equal to 2.5%, formoterol fumarate, budesonide are preferably combined in a single dosage form.

Furthermore, the amount of active agents used in the composition is carefully adjusted in order to prevent the side effects that might arise from these agents and it was seen that minimum side effects are observed when tiotropium bromide with water content less than or equal to 2.5%: formoterol fumarate: budesonide ratio in the composition is in the range of 1 : 0.1 : 2 to 1 : 3 : 40 by weight. Furthermore, it was seen that in the formulation, that is constituted with active agents which have ratios in the range of 1 : 0,1 : 2 to 1 : 3 : 40 , the adhesive force between the particles is less and hence the amount of inhaled particles and efficacy of the formulation increases.

Accordingly, the present invention provides a combined drug preparation comprising tiotropium bromide with water content less than or equal to 2.5%. formoterol fumarate and budesonide in amounts in the range of 1 : 0,1 : 2 to 1 : 3 : 40 for simultaneous or sequential administration in the prevention or treatment of respiratory diseases such as asthma and chronic obstructive pulmonary diseases (COPD).

According to another aspect, the present invention provides an effective inhalation of the drug comprising tiotropium bromide with water content less than or equal to 2.5%, formoterol fumarate and budesonide.

The drug pertaining to the present invention is preferred to be administered by the inhalation route as it a) has a more rapid onset of action compared to the administration via oral or parenteral routes b) enables the use at lower doses c) minimizes the side effects.

According to another aspect, the present invention provides simultaneous or sequential inhalation of the drug comprising tiotropium bromide with water content less than or equal to 2.5%, formoterol fumarate and budesonide by the inhalation route.

According to another aspect, the present invention provides the transmission of the drug comprising tiotropium bromide with water content less than or equal to 2.5%, formoterol fumarate and budesonide via single or multi dose inhalers.

The drug comprising tiotropium bromide with water content less than or equal to 2.5%, formoterol fumarate and budesonide can be in dry powder form; they can be formulated with propellant gases to give aerosol formulations or they can be formulated with solvents to give nebulizer formulations. The inventors have found that the best way to transfer the medicament comprising tiotropium bromide with water content less than or equal to 2.5%, formoterol fumarate and budesonide is by using this medicament combination in dry powder form.

In this way, the components maintain their stability and furthermore, the medicament is in a stable form and is easily used by the patients.

In order to ensure effective absorption of the active agents into the lung tissue, the particle size of the agents should be adjusted. Although large particle size provides ease in manufacturing the dry powder, it may accumulate in throat and lead to insufficient intake of the medicament. Very fine particles, on the other hand, may reach the lungs. However, they might not have a good flow property which causes problems in providing dose accuracy in turn. To prevent these problems, the active agents should have an optimum average particle size. The inventors have found that tiotropium bromide with water content less than or equal to 2.5%, formoterol fumarate, budesonide having a mean particle size in the range of 1.5 to 4.5 µm reaches the lungs effectively and also no problems related to flow properties of the dry powder are observed.

In one aspect, the present invention provides a medicament composition comprising tiotropium bromide with water content less than or equal to 2.5%, formoterol fumarate and budesonide in dry powder form wherein the mean particle size of tiotropium bromide with water content less than or equal to 2.5%, formoterol fumarate and budesonide is in the range of 1.5 to 4.5 µm and wherein said active agents are present in the composition with the ratio of 1 : 0,1 : 2 to 1 : 3 : 40 respectively and said composition can be simultaneously or sequentially administered in the prevention or treatment of respiratory diseases.

The term "mean particle size" refers to particles wherein the particle size of 50% of the total number of particles is less than the average particle size.

According to the present invention, the drug comprising tiotropium bromide with water content less than or equal to 2.5%, formoterol fumarate and budesonide may also contain effective amounts of excipients and/or additional agents apart from active agents.

According to the present invention, the dry powder formulation comprising tiotropium bromide with water content less than or equal to 2.5%, formoterol fumarate and budesonide is transmitted to the patient in dry powder form. Said dry powder formulations also contain some physiologically acceptable excipients along with the active agent. These excipients can be monosaccharides (glucose, etc.), disaccharides (lactose, saccharose, maltose, etc.), oligosaccharides and polysaccharide (dextran, etc.), polyalcohols (sorbitol, mannitol, xylitol, etc.), salts (sodium chloride, calcium carbonate, etc.) or a mixture thereof.

The inventors have found that in the compositions pertaining to the present invention, in other words in the compositions comprising tiotropium bromide with water content less than or equal to 2.5%, formoterol fumarate and budesonide in dry powder form wherein the mean particle size of tiotropium bromide with water content less than or equal to 2.5%, formoterol fumarate and budesonide is in the range of 1.5 to 4.5 µm and wherein said active agents are present in the composition with a ratio of 1 : 0,1 : 2 to 1 : 3 : 40 respectively, using lactose as the one and only carrier provides optimum homogeneity and flow properties to the dry powder and therefore, dose accuracy is maintained.

It was also seen that the mean particle size of the carrier plays an important role in the delivery of the medicament to the target area, i.e. lungs, effectively in the compositions pertaining to the present invention. It was found that the adhesive forces present between the lactose particles and the active agents having a mean particle size in the range of 1.5 to 4.5 µm are minimized and thus, an effective inhalation of the active agents takes place when lactose having a mean particle size less than or equal to 100 µm is used in the compositions comprising tiotropium bromide with water content less than or equal to 2.5%, fonnoterol fumarate and budesonide in dry powder form wherein the mean particle size of tiotropium bromide with water content less than or equal to 2.5%, formoterol fumarate and budesonide is in the range of 1.5 to 4.5 µm and wherein said active agents are present in the composition with the ratio of 1 : 0.1 : 2 to 1 : 3 : 40 respectively.

In another aspect, the present invention provides a composition comprising tiotropium bromide with water content less than or equal to 2.5%, formoterol fumarate and budesonide in dry powder form wherein;
- the mean particle size of tiotropium bromide with water content less than or equal to 2.5%, formoterol fumarate and budesonide is in the range of 1.5 to 4.5 µm and
- said active agents are present in the composition with the ratio of 1 : 0,1 : 2 to 1 : 3 : 40 respectively and
- lactose which has a mean particle size less than 100 µm is used as carrier.

Lactose which has a mean particle size less than 100 µm is preferably used as a mixture of particles having two different mean particle sizes. Accordingly, lactose which has a mean particle size less than 100 µm can be present as a mixture of particles having a mean particle size less than 10 µm (fine) and particles having a mean particle size in the range of 10 µm to 100 µm (coarse). The inventors have observed that the adhesive force between the active agents and lactose is even less when lactose which has two different mean particle sizes is used.

The ratio of lactose which has a mean particle size less than 10 µm (fine) to lactose which has a mean particle size in the range of 10 µm to 100 µm (coarse) is in the range of 1:1 to 1:25, preferably in the range of 1:1 to 1:10, most preferably in the range of 1 : 1,5 to 1: 5.

According to the present invention, the amount of pharmaceutically acceptable carrier is preferably in the range of up to 50 mg.

According to another aspect, the present invention provides a method to transmit the drug combination comprising tiotropium, formoterol and budesonide and/or pharmaceutically acceptable derivatives thereof via a dry powder inhaler in which the drug is stored in peelable blister packs, capsules or a reservoir for use in the treatment of patients suffering from respiratory diseases.

In the inhalation devices which are designed to transmit dry powder drugs, an effective amount of the dry powder drug is prepared for inhalation when the device is triggered.

In order to prepare the dry powder formulation stored in capsules, the supplementary components in the device provides the capsule to open or be pierced when the device is triggered and the dry powder formulation is prepared for inhalation. After the inhalation is completed, the empty capsule is ejected from the device and a new capsule is placed immediately before the following inhalation takes place.

According to the present invention, the capsule can be made of a substance chosen from a group comprising gelatine, chitosan, starch and/or starch derivatives, cellulose and/or cellulose derivatives or synthetic polymers as well as consisting intertwined upper and lower compartments.

In the case that the capsule used in the present invention is made of cellulose or its derivatives, the capsule material can be selected from, but not limited to, a group comprising hydroxypropyl cellulose, hydroxypropylmethyl cellulose, methyl cellulose, hydroxymethyl cellulose, hydroxyethyl cellulose.

In the case that the capsule used in the present invention is synthetic polymer, the capsule material can be selected from, but not limited to, a group comprising polyethylene, polyethyleneteraphtalate, polycarbonate or polypropylene.

In the case that the capsule material used in the present invention is gelatine, additional agents such as polyethylene glycol, sorbitol, glycerol, propylene glycol, polyethylene oxide - polypropylene oxide block copolymers and/or other polyalcohols or polyethers at different molecular weights can be added into it.

The dry powder drug pertaining to the present invention can also be stored in blister packs apart from reservoirs and capsules. Blister packs are comprised of orderly placed blisters each of which contains minimally one dose of the dry powder drug. Blister packs can be pierced or peeled to be opened according to the device design. However, peelable blister packs are preferred according to the present invention. When the device is triggered, the blister pack or one of the blisters in the pack is pierced or peeled and the drug in dry powder form is prepared for inhalation.

The cavity volume of blisters pertaining to the present invention, which arc placed side by side in an order and which provide to transmit and store the dry powder drug in blister pack is in the range of 17-30 mm³, preferably in the range of 18-23 mm³, most preferably in the range of 19-21 mm³

The cavity volume of the blisters pertaining to the present invention, which provide to transmit and store the dry powder drug comprising tiotropium bromide having water content less than or equal to 2.5%, formoterol fumarate and budesonide is in the range of 17-30 mm³, preferably in the range of 18-23 mm³, most preferably in the range of 19-21 mm³ and each blister cavity having the volume described above is filled up to 25-100 %, preferably up to 70-100 %, most preferably up to 90-100 % of said volume in order to meet the specified needs for an effective inhalation. The lid and the base sheets of said blister pack are closed very tightly by any suitable method to provide impermeability.

According to the present invention, the lid and the base sheet constituting the blister package consist of several layers. Polymeric layers, aluminum foil and preferably Aclar® fluoropoylmer film are among the layers that form the lid and the base sheet.

Aclar® fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

In order to decrease the gas and moisture permeability of the layer, preferably desiccant agents are added to the polymeric layers to preserve the stability of the dry powder formulation stored in blisters that are arranged in an order on blister strips. Silica gel, zeolite. alumina, bauxite, anhydrous calcium sulfate, activated carbon and clay which have the property of water absorption can be given as examples to desiccant agents.

As it is common to use aluminum in lid and base sheets of high protection blister packs, aluminum is used both in the lid and the base sheets of the blister pack of the present invention in order to provide high moisture and gas protection. These aluminum foils must be thick enough to provide the desired protection for the stability of the moisture sensitive dry powder formulation stored in the blister cavity. Due to this reason, the thickness of the aluminum foil that is used in the lid and the base sheets of the blister pack is chosen to be in the range of 10 to 40 µm, preferably of 15 to 30 µm.

The polymeric layers in the lid and the base sheets of the blister pack mentioned in the present invention are made from the same or different polymers. The thickness of these polymeric layers varies according to the type of the polymeric substance used and its properties. Therefore, the thickness of the polymeric layer varies in the range of 15-60 µm, preferably of 20-35 µm depending on the type of the polymer used.

The inside layer of the blister cavity of the said blister pack which is in contact with the dry powder formulation is a polymeric layer because of the fact that some of the dry powder formulation sticks onto the inside layer of the blister cavity due to the porous structure of aluminum foil and electrostatic forces, and hence causes uncontrolled dosing.

According to the present invention, the polymers used to form the polymeric layers are preferably selected from a group comprising thermo-plastic polymers such as polyethylene, polypropylene, polystyrene, polyolefin, polyamide, polyvinyl chloride, polyurethane or other synthetic polymers.

In addition, the blisters which constitute the blister pack pertaining to the present invention can be in any shape as long as they have the properties described above.

According to the present invention, tiotropium bromide with water content less than or equal to 2.5% can be in crystal form and/or amorphous form or combination thereof.

According to the present invention, formoterol fumarate can be in the form of its solvates, hydrates enantiomers, diastereomers, racemates and/or in crystal form and/or amorphous form and/or a combination thereof.

According to the present invention, budesonide can be in the form of its solvates, hydrates, enantiomers or diastereoisomers, racemates and/or in crystal form and/or in amorphous forms and/or a combination thereof.

According to the present invention, the amount of tiotropium bromide water content less than or equal to 2.5% included in the drug formulation comprising tiotropium bromide with water content less than or equal to 2.5%, formoterol fumarate and budesonide is in the range of 1-40 µg, preferably 1-30 µg per dose.

According to the present invention, the amount of formoterol fumarate included in the drug formulation comprising tiotropium bromide with water content less than or equal to 2.5%, formoterol fumarate and budesonide is in the range of 1-30µg, preferably 1-20 µg per dose.

According to the present invention, the amount of budesonide included in the drug formulation comprising tiotropium bromide with water content less than or equal to 2.5%, formoterol fumarate and budesonide is in the range of 20-600 µg, preferably 50-500 µg per dose.

The pharmaceutical composition mentioned in the present invention which comprises tiotropium bromide with water content less than or equal to 2.5%, formoterol fumarate and budesonide in dry powder form wherein;
- the particle size of tiotropium bromide with water content less than or equal to 2.5%, formoterol fumarate and budesonide is in the range of 1.5 to 4.5 µm and
- said active agents are present in the composition with the ratio of 1 : 0,1 : 2 to 1 : 3 : 40 respectively and
- lactose which has a mean particle size less than 100 µm is used as carrier
can be used in the treatment of many respiratory diseases such as asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition, the pharmaceutical composition pertaining to the present invention is used especially for the symptomatic treatment of asthma, allergic rhinitis and COPD.

A method for preparing the pharmaceutical composition according to the present invention comprises micronizing the tiotropium bromide with water content less than or equal to 2.5%, formoterol fumarate and budesonide, preferably by air jet mill, mixing the micronized tiotropium bromide with water content less than or equal to 2.5%, formoterol fumarate and budesonide with lactose, then blending the composition to obtain a homogeneous dry powder mixture, and then filling the obtained dry powder mixture into capsules or blisters.

The combination of the pharmaceutical composition pertaining to the present invention can be explained with, but not restricted to, the examples given below.

### Example 1

So as to be used in a multiple dose inhaler, a dry powder drug formulation which is appropriate to be stored in blisters comprises 18 parts of tiotropium bromide with water content less than or equal to 2.5%, 12 parts of formoterol fumarate, 200 parts of budesonide having a mean particle size of 1.5-4.5 µm all of which are micronized in air jet mill and 10000 parts of lactose as carrier which has a particle diameter less than 100 µm.

The active substance tiotropium bromide with water content less than or equal to 2.5% given in this example includes its pharmaceutically acceptable amorphous and crystal forms thereof, formoterol fumarate includes its all pharmaceutically acceptable racemates, enantiomers or diastereomers, solvates, hydrates and/or amorphous and crystal forms and budesonide includes its all pharmaceutically acceptable solvates, hydrates and/or enantiomers and/or amorphous and crystal forms. Lactose in this example can optionally be added more or less.

The amounts in Example 1 can be replaced by the amounts given in the table below and the example can be repeated.

| EXAMPLE | Amount of Tiotropium Bromide (parts) | Amount of Formoterol Fumarate (parts) | Amount of Budesonide (parts) | Amount of Lactose (parts) |
|---|---|---|---|---|
| 2 | 11 | 12 | 200 | 11250 |
| 3 | 18 | 12 | 200 | 13750 |
| 4 | 18 | 12 | 200 | 14000 |
| 5 | 11 | 12 | 200 | 15260 |
| 6 | 11 | 12 | 200 | 15300 |
| 7 | 21 | 12 | 200 | 14680 |
| 8 | 11 | 6 | 400 | 16800 |
| 9 | 11 | 6 | 400 | 15500 |
| 10 | 21 | 9 | 400 | 13690 |
| 11 | 11 | 12 | 200 | 14890 |
| 12 | 11 | 12 | 200 | 16100 |
| 13 | 21 | 12 | 400 | 16930 |
| 14 | 11 | 6 | 400 | 16750 |
| 15 | 11 | 6 | 400 | 12530 |
| 16 | 21 | 12 | 200 | 13590 |
| 17 | 11 | 12 | 400 | 17500 |
| 18 | 11 | 12 | 400 | 15500 |
| 19 | 18 | 12 | 400 | 16500 |

### Example 20

A dry powder formulation which is appropriate for a gelatine capsule used in a capsule inhaler comprises 18 parts of tiotropium bromide with water content less than or equal to 2.5%, 12 parts of formoterol fumarate, 200 parts of budesonide having a mean particle size of 1,5 - 4,5 µm all of which are micronized in air jet mill and 5500 parts of lactose as carrier which has a particle diameter less than 100 µm.

The active agent tiotropium bromide with water content less than or equal to 2.5% given in this example includes its all pharmaceutically acceptable amorphous and/or crystal forms thereof; formoterol fumarate includes its all pharmaceutically acceptable racemates, enantiomers or diastereomers, solvates, hydrates and/or amorphous and/or crystal forms and budesonide includes its all pharmaceutically acceptable solvates, hydrates and/or enantiomers, and/or amorphous and/or crystal forms. Lactose given in this example can optionally be added more or less. The capsule in this example is made of gelatin but it can optionally be made of chitosan, starch and/or starch derivatives, cellulose and/or cellulose derivatives or synthetic polymers.

The combination of the pharmaceutical composition pertaining to the present invention can be explained with, but not restricted to, the examples given below.

The amounts used in Example 20 can be replaced by the amounts given in the table below and the example can be repeated.

| EXAMPLE | Amount of Tiotropium Bromide (parts) | Amount of Formoterol Fumarate (parts) | Amount of Budesonide (parts) | Amount of Lactose (parts) |
|---|---|---|---|---|
| 21 | 11 | 12 | 200 | 4250 |
| 22 | 11 | 12 | 200 | 5230 |
| 23 | 11 | 12 | 200 | 4750 |
| 24 | 21 | 6 | 400 | 5500 |
| 25 | 21 | 6 | 400 | 5000 |
| 26 | 21 | 6 | 400 | 6100 |
| 27 | 21 | 6 | 200 | 4000 |
| 28 | 21 | 12 | 400 | 6350 |
| 29 | 11 | 12 | 200 | 5720 |
| 30 | 11 | 12 | 200 | 4720 |
| 31 | 11 | 12 | 200 | 426 0 |
| 32 | 11 | 12 | 200 | 5200 |
| 33 | 11 | 6 | 400 | 5500 |
| 34 | 21 | 6 | 200 | 4600 |
| 35 | 21 | 12 | 400 | 4810 |
| 36 | 11 | 12 | 400 | 6000 |
| 37 | 11 | 12 | 400 | 5420 |
| 38 | 21 | 12 | 400 | 4950 |

## Claims

1. A pharmaceutical composition for use in the simultaneous or sequential administration in the symptomatic and/or prophylactic treatment of respiratory diseases comprising tiotropium bromide with water content less than or equal to 2.5%, formoterol fumarate and budesonide in dry powder form wherein,
∘ the mean particle size of tiotropium bromide with water content less than or equal to 2.5%, formoterol fumarate and budesonide is in the range of 1.5 to 4.5 µm and
∘ said active agents are present in the composition with the ratio of 1 : 0,1 : 2 to 1 : 3 : 40 respectively and
∘ lactose which is used as carrier has a mean particle size less than 100 µm.

2. The pharmaceutical composition for use according to claim 1, wherein said composition comprises tiotropium bromide with water content less than or equal to 2.5%, formoterol fumarate, budesonide and lactose.

3. The pharmaceutical composition for use according to claim 1, wherein tiotropium bromide with water content less than or equal to 2.5%, formoterol fumarate, budesonide are combined in a single dosage form.

4. The pharmaceutical composition for use according to claim 1, wherein the amount of formoterol fumarate and/or its pharmaceutically acceptable solvates, hydrates enantiomers, diastereomers, racemates is in the range of 1-30 µg per dose.

5. The pharmaceutical composition for use according to claim 1, wherein the formoterol fumarate is dihydrate.

6. The pharmaceutical composition for use according to claim 1, wherein the amount of budesonide and/or its pharmaceutically acceptable solvates, hydrates, enantiomers or diastereoisomers, racemates is in the range of 20-600 µg per dose.

7. The pharmaceutical composition for use according to claim 1, wherein the amount of tiotropium bromide with water content less than or equal to 2.5% is in the range of 1-40 µg per dose.

8. The pharmaceutical composition for use according to claim 1, wherein lactose which has a mean particle size less than 100 µm is used as a mixture of particles having two different mean particle sizes.

9. The pharmaceutical composition for use according to claim 8, wherein lactose which has a mean particle size less than 100 µm is present as a mixture of particles having a mean particle size less than 10 µm and particles having a mean particle size in the range of 10 µm to 100 µm.

10. The pharmaceutical composition for use according to claim 9, wherein the ratio of lactose which has a mean particle size less than 10 µm (fine) to lactose which has a mean particle size in the range of 10 µm to 100 µm (coarse) is in the range of 1:1 to 1:25.

11. The pharmaceutical composition for use according to claim 9, wherein the ratio of lactose which has a mean particle size less than 10 µm (fine) to lactose which has a mean particle size in the range of 10 µm to 100 µm (coarse) is in the range of 1:1 to 1:10.

12. The pharmaceutical composition for use according to claim 9, wherein the ratio of lactose which has a mean particle size less than 10 µm (fine) to lactose which has a mean particle size in the range of 10 µm to 100 µm (coarse) is in the range of 1:1,5 to 1:5.

13. The pharmaceutical composition for use according to claim 1. wherein the amount of lactose is in the range of up to 50 mg.

14. A pharmaceutical composition comprising tiotropium bromide with water content less than or equal to 2.5%, formoterol fumarate and budesonide in dry powder form wherein ∘
∘ the mean particle size of tiotropium bromide with water content less than or equal to 25%, formoterol fumarate and budesonide is in the range of 1.5 to 4.5 µm and
∘ said active agents are present in the composition with the ratio of 1 : 0,1 : 2 to 1 : 3 : 40 respectively and
∘ lactose which is used as carrier has a mean particle size less than 100 µm
is administered simultaneously or sequentially for use in treatment of respiratory diseases especially asthma, allergic rhinitis, chronic obstructive pulmonary disease (COPD).

15. The pharmaceutical composition for use according to claim 1, wherein said composition is used for simultaneous administration in the symptomatic and/or prophylactic treatment of respiratory diseases.

## Patentansprüche

1. Pharmazeutische Zusammensetzung, die zur gleichzeitigen oder sequentiellen Verabreichung bei der symptomatischen und/oder prophylaktischen Behandlung von Atemwegserkrankungen verwendet wird, umfassend Tiotropiumbromid mit einem Wassergehalt von weniger als oder gleich 2.5%, Formoterolfumarat und Budesonid in Trockenpulverform, wobei,
o die mittlere Partikelgröße von Tiotropiumbromid mit einem Wassergehalt von weniger als oder gleich 2.5%, Formoterolfumarat und Budesonid im Bereich von 1.5 bis 4.5 µm liegt und
∘ die Wirkstoffe in der Zusammensetzung im Verhältnis von jeweils 1 : 0,1 : 2 bis 1:3: 40 vorhanden sind und
∘ Laktose, die als Träger verwendet wird, eine mittlere Partikelgröße von weniger als 100 µm aufweist.

2. Pharmazeutische Zusammensetzung zur Verwendung nach Anspruch 1, wobei die Zusammensetzung Tiotropiumbromid mit einem Wassergehalt von weniger als oder gleich 2.5% Formoterolfumarat, Budesonid und Laktose umfasst.

3. Pharmazeutische Zusammensetzung zur Verwendung nach Anspruch 1, wobei Tiotropiumbromid mit einem Wassergehalt von weniger als oder gleich 2.5%, Formoterolfumarat, Budesonid in einer einzigen Dosierungsform kombiniert sind.

4. Pharmazeutische Zusammensetzung zur Verwendung nach Anspruch 1, wobei die Menge an Formoterolfumarat und/oder seinen pharmazeutisch verträglichen Solvaten, Hydraten, Enantiomeren, Diastereomeren und Racematen im Bereich von 1-30 µg pro Dosis liegt.

5. Pharmazeutische Zusammensetzung zur Verwendung nach Anspruch 1, wobei das Formoterolfumarat Dihydrat ist.

6. Pharmazeutische Zusammensetzung zur Verwendung nach Anspruch 1, wobei die Menge an Budesonid und/oder seinen pharmazeutisch verträglichen Solvaten, Hydraten, Enantiomeren oder Diastereoisomeren, Racematen im Bereich von 20-600 µg pro Dosis liegt.

7. Pharmazeutische Zusammensetzung zur Verwendung nach Anspruch 1, wobei die Menge an Tiotropiumbromid mit einem Wassergehalt von weniger als oder gleich 2.5% im Bereich von 1-40 µg pro Dosis liegt.

8. Pharmazeutische Zusammensetzung zur Verwendung nach Anspruch 1, wobei Laktose, die eine mittlere Partikelgröße von weniger als 100 µm aufweist, als Gemisch von Partikeln mit zwei verschiedenen mittleren Partikelgrößen verwendet wird.

9. Pharmazeutische Zusammensetzung zur Verwendung nach Anspruch 8, wobei Laktose, die eine mittlere Partikelgröße von weniger als 100 µm aufweist, als eine Gemisch von Partikeln mit einer mittleren Partikelgröße von weniger als 10 µm und Partikeln mit einer mittleren Partikelgröße im Bereich von 10 µm bis 100 µm vorliegt.

10. Pharmazeutische Zusammensetzung zur Verwendung nach Anspruch 9, wobei das Verhältnis von Laktose, die eine mittlere Partikelgröße von weniger als 10 µm (fein) aufweist, zu Laktose, die eine mittlere Partikelgröße im Bereich von 10 µm bis 100 µm (grob) aufweist, im Bereich von 1:1 bis 1:25 liegt.

11. Pharmazeutische Zusammensetzung zur Verwendung nach Anspruch 9, wobei das Verhältnis von Laktose, die eine mittlere Partikelgröße von weniger als 10 µm (fein) aufweist, zu Laktose, die eine mittlere Partikelgröße im Bereich von 10 µm bis 100 µm (grob) aufweist, im Bereich von 1:1 bis 1:10 liegt.

12. Pharmazeutische Zusammensetzung zur Verwendung nach Anspruch 9, wobei das Verhältnis von Laktose, die eine mittlere Partikelgröße von weniger als 10 µm (fein) aufweist, zu Laktose, die eine mittlere Partikelgröße im Bereich von 10 µm bis 100 µm (grob) aufweist, im Bereich von 1:1,5 bis 1:5 liegt.

13. Pharmazeutische Zusammensetzung zur Verwendung nach Anspruch 1, wobei die Menge an Laktose im Bereich von 0,01-50 µg liegt.

14. Pharmazeutische Zusammensetzung, umfassend Tiotropiumbromid mit einem Wassergehalt von weniger als oder gleich 2.5%, Formoterolfumarat und Budesonid in Trockenpulverform, wobei
∘ die mittlere Partikelgröße von Tiotropiumbromid mit einem Wassergehalt von weniger als oder gleich 2.5%, Formoterolfumarat und Budesonid im Bereich von 1.5 bis 4.5 µm liegt und
∘ die Wirkstoffe in der Zusammensetzung mit dem Verhältnis von jeweils 1 : 0,1 : 2 bis 1 : 3 : 40 vorhanden sind und
∘ Laktose, die als Träger verwendet wird, eine mittlere Partikelgröße von weniger als 100 µm aufweist.
wird gleichzeitig oder sequentiell zur Behandlung von Atemwegserkrankungen, insbesondere Asthma, allergischer Rhinitis, chronisch obstruktiver Lungenerkrankung (COPD), verabreicht.

15. Pharmazeutische Zusammensetzung zur Verwendung nach Anspruch 1, wobei die Zusammensetzung zur gleichzeitigen Verabreichung bei der symptomatischen und/oder prophylaktischen Behandlung von Atemwegserkrankungen verwendet wird.

## Revendications

1. Composition pharmaceutique utilisée pour une administration simultanée ou séquentielle dans le traitement symptomatique et/ou prophylactique de maladies respiratoires comprenant du bromure de tiotropium avec une teneur en eau inférieure ou égale à 2,5%, fumarate de formotérol et budésonide sous forme de poudre sèche dans laquelle,
∘ la taille moyenne des particules de bromure de tiotropium ayant une teneur en eau inférieure ou égale à 2,5 %, de fumarate de formotérol et de budésonide est comprise entre 1,5 et 4,5 µm et
∘ lesdits agents actifs sont présents dans la composition avec un rapport de 1 : 0,1 : 2 à 1:3: 40 respectivement et
∘ le lactose utilisé comme support a une granulométrie moyenne inférieure à 100 µm.

2. Composition pharmaceutique pour utilisation selon la revendication 1, dans laquelle ladite composition comprend du bromure de tiotropium ayant une teneur en eau inférieure ou égale à 2,5 %. fumarate de formotérol, budésonide et lactose.

3. Composition pharmaceutique pour utilisation selon la revendication 1, dans laquelle le bromure de tiotropium ayant une teneur en eau inférieure ou égale à 2,5 %, le fumarate de formotérol et le budésonide sont combinés dans une seule forme posologique.

4. Composition pharmaceutique pour utilisation selon la revendication 1, dans laquelle la quantité de fumarate de formotérol et/ou de ses solvates pharmaceutiquement acceptables, d'énantiomères d'hydrates, de diastéréoisomères, de racémates est dans l'intervalle de 1-30 µg par dose.

5. Composition pharmaceutique pour utilisation selon la revendication 1, dans laquelle le fumarate de formotérol est du dihydrate.

6. Composition pharmaceutique pour utilisation selon la revendication 1, dans laquelle la quantité de budésonide et/ou de ses solvates, hydrates, énantiomères ou éoisomères diastériques, racémates ou racémates pharmaceutiquement acceptables est comprise entre 20 et 600 µg par dose.

7. Composition pharmaceutique pour utilisation selon la revendication 1, dans laquelle la quantité de bromure de tiotropium ayant une teneur en eau inférieure ou égale à 2,5 % est comprise entre 1-40 µg par dose.

8. Composition pharmaceutique pour utilisation selon la revendication 1, dans laquelle le lactose qui a une taille moyenne de particules inférieure à 100 µm est utilisé comme mélange de particules ayant deux tailles moyennes de particules différentes.

9. Composition pharmaceutique pour utilisation selon la revendication 8, dans laquelle le lactose qui a une taille moyenne de particules inférieure à 100 µm est présent sous la forme d'un mélange de particules ayant une taille moyenne de particules inférieure à 10 µm et de particules ayant une taille moyenne de particules comprise entre 10 µm à 100 µm.

10. Composition pharmaceutique pour utilisation selon la revendication 9, dans laquelle le rapport du lactose qui a une taille moyenne de particules inférieure à 10 µm (fine) au lactose qui a une taille moyenne de particules dans la gamme de 10 µm à 100 µm (grossière) est dans la gamme de 1:1 à 1:25.

11. Composition pharmaceutique pour utilisation selon la revendication 9, dans laquelle le rapport du lactose qui a une taille moyenne de particules inférieure à 10 µm (fine) au lactose qui a une taille moyenne de particules dans la gamme de 10 µm à 100 µm (grossière) est dans la gamme de 1:1 à 1:10.

12. Composition pharmaceutique pour utilisation selon la revendication 9, dans laquelle le rapport du lactose qui a une taille moyenne de particules inférieure à 10 µm (fine) au lactose qui a une taille moyenne de particules dans la gamme de 10 µm à 100 µm (grossière) est dans la gamme de 1:1,5 à 1:5.

13. Composition pharmaceutique pour utilisation selon la revendication 1, dans laquelle la quantité de lactose est comprise entre 0,01- 50 mg.

14. Composition pharmaceutique comprenant du bromure de tiotropium ayant une teneur en eau inférieure ou égale à 2,5 %, du fumarate de formotérol et du budésonide sous forme de poudre sèche dans laquelle
∘ la taille moyenne des particules de bromure de tiotropium ayant une teneur en eau inférieure ou égale à 2,5 %, de fumarate de formotérol et de budésonide est comprise entre 1,5 à 4,5 µm et
∘ lesdits agents actifs sont présents dans la composition avec un rapport de 1 : 0,1 : 2 à 1 : 3 : 40 respectivement et
∘ le lactose qui est utilisé comme support a une taille moyenne de particules inférieure à 100 µm
est administré simultanément ou séquentiellement pour le traitement des maladies respiratoires, en particulier l'asthme, la rhinite allergique, la maladie pulmonaire obstructive chronique (MPOC).

15. Composition pharmaceutique pour utilisation selon la revendication 1, dans laquelle ladite composition est utilisé pour l'administration simultanée dans le traitement symptomatique et/ou prophylactique des maladies respiratoires.
